# EUROPEAN PATENT SPECIFICATION

(11) **EP 1 527 199 B1**
(45) Date of publication and mention of the grant of the patent: **07.07.2010**
(21) Application number: 03746704.0
(22) Date of filing: 11.04.2003
(51) Int. Cl.: C12Q 1/68, C12P 19/34, C07H 21/04

(54) **METHODS FOR DETECTING ABNORMALLY METHYLATED NUCLEIC ACID IN HETEROGENEOUS BIOLOGICAL SAMPLES**
VERFAHREN ZUM NACHWEIS ABNORMAL METHYLIERTER NUKLEINSÄURE IN HETEROGENEN BIOLOGISCHEN PROBEN
PROCEDES DE DETECTION D'ACIDE NUCLEIQUE ANORMALEMENT METHYLE DANS DES ECHANTILLONS BIOLOGIQUES HETEROGENES

(30) Priority: 11.04.2002 US 123071
(43) Date of publication of application: 04.05.2005
(73) Proprietor: Genzyme Corporation, Cambridge, MA 02142 (US)
(72) Inventor: SHUBER, Anthony, P., Mendon, MA 01756 (US)
(74) Representative: Aston, Heidi Frances
(86) International application number: PCT/US2003/011023
(87) International publication number: WO 2003/087390

(56) References cited:
- WO-A-00/26401
- WO-A-01/68911
- WO-A-01/75172
- WO-A-02/18649
- WO-A-99/55905
- US-A1- 2002 025 525
- US-B1- 6 207 372
- US-B1- 6 268 136
- NAGASAKA T ET AL: "CPG ISLAND MICROARRAY TO ANALYZE METHYLATION PROFILES OF O6 METHYLGUANINE-DNA METHYLTRANSFERASE IN COLON CANCER" PROCEEDINGS OF THE ANNUAL MEETING OF THE AMERICAN ASSOCIATION FOR CANCER RESEARCH, NEW YORK, NY, US, vol. 43, March 2002 (2002-03), page 1129, XP001188170 ISSN: 0197-016X
- MODEL F ET AL: "FEATURE SELECTION FOR DNA METHYLATION BASED CANCER CLASSIFICATION" BIOINFORMATICS, OXFORD UNIVERSITY PRESS, OXFORD,, GB, vol. 17, no. SUPPL 1, 2001, pages 157-164, XP001154594 ISSN: 1367-4803
- BEVILACQUA R A U ET AL: "Methylation of the hMLH1 promoter but not hMLH1 mutations sporadic gastric carcinomas with high-level microsatellite instability" INTERNATIONAL JOURNAL OF CANCER, NEW YORK, NY, US, vol. 87, January 2000 (2000-01), pages 200-203, XP002960987 ISSN: 0020-7136
- MOINOVA H R ET AL: "HLTF gene silencing in human colon cancer" PROCEEDINGS OF THE NATIONAL ACADEMY OF SCIENCES OF USA, NATIONAL ACADEMY OF SCIENCE, WASHINGTON, DC, US, vol. 99, no. 7, 2 April 2002 (2002-04-02), pages 4562-4567, XP002347365 ISSN: 0027-8424
- HILTUNEN M O ET AL: "Hypermethylation of the APC (adenomatous polyposis coli) gene promoter region in human colorectal carcinoma" INTERNATIONAL JOURNAL OF CANCER, NEW YORK, NY, US, vol. 70, no. 6, 17 March 1997 (1997-03-17), pages 644-648, XP002305016 ISSN: 0020-7136
- HERMAN ET AL.: 'METHYLATION-SPECIFIC PCR: A NOVEL PCR ASSAY FOR METHYLATION STATUS OF CPG ISLANDS' PROC. NATL. ACAD. SCI. USA vol. 93, 01 September 1996, pages 9821 - 9826, XP002910406
- SHUBER A. P. ET AL.: 'A SIMPLIFIED PROCEDURE FOR DEVELOPING MULTIPLEX PCRS' GENOME RESEARCH vol. 5, no. 5, 01 December 1995, pages 488 - 493, XP000611203

## Description

### Field of the Invention

The invention relates generally to methods for detecting indicia of cancer in biological samples. In particular, the invention relates to methods for detecting the presence of hypermethylated nucleic acids in biological samples.

### Background of the Invention

In higher order eukaryotes, DNA may be methylated at cytosines located 5' to guanosine in CpG dinucleotides. This modification has important regulatory effects on gene expression, especially when involving CpG rich areas, known as CpG islands (typically at least 50% GC content in a 200 base pairs (bps) long nucleic acid sequence) and often found in the promoter regions of many genes. Aberrant methylation of normally unmethylated CpG islands has been associated with transcriptional inactivation of defined tumor suppressor genes in human cancers, e.g., colorectal cancer (CRC). Other types of methylation changes such as loss of imprinting in certain genes resulting from hypomethylation has also been linked to a higher risk of a variety of cancers including CRC. See, Cui et al. "Science," vol. 299, 1753-55 (March 14, 2003). Therefore, detection of changes in nucleic acid methylation can indicate the onset of or a higher risk for various forms of cancers.

Various methods have been described for detecting methylation changes at specific loci on genomic DNA. One of these methods involves modification of DNA by sodium bisulfite or a comparable agent which converts all unmethylated cytosines to uracils without converting methylated cytosines, and subsequent amplification with primers specific for methylated versus unmethylated DNA. However, this and other hypermethylation detection methods require homogeneous nucleic acid samples that have a sufficient amount of the target nucleic acid. For many types of cancers, invasive procedures such as surgery are needed to obtain such a homogenous sample.

However, many biological samples, such as stool samples, are heterogeneous and may contain a small amount of mutant nucleic acid mixed with a large amount of wild-type nucleic acid. Therefore, there is a need in the art for methods to detect small amounts of hypermethylation in heterogeneous biological samples.
US 2002/025525 discloses methods for detecting contamination in molecular diagnostics using PCR.
WO 00/26401 relates to a method of determining the DNA methylation status of CpG sites in a given locus.
Nagasaka et al. Proceedings of the Annual Meeting of the American Association for Cancer Research, New York, NY, US, Vol. 43, March 2002, p.1129, discloses a CpG island microarray to analyze methylation profiles of 06 methylguanine-DNA methyltransferase in colon cancer.
WO 01/75172 discloses a diagnostic or prognostic assay for gastrointestinal adenocarcinoma.
WO 02/18649 discloses a nested methylation specific polymerase chain reaction cancer detection method.
Bevilacqua et al, International Journal of Cancer, NY, US, Vol. 87, January 2000, pages 200-203, discloses the methylation of the *hMLH1* promoter but no *hMLH1* mutations in sporadic gastric carcinomas with high-level microsatellite instability.
Moinova et al, PNAS Vol. 99, no. 7, 2 April 2002, pages 4562-4567 relates to HLTF gene silencing in human colon cancer.
Hiltunen et al, International Journal of Cancer, NY, US, Vol. 70. no. 6, 17 March 1997, pages 644-648, relates to the hypermethylation of the APC (adenomatous polyposis coli) gene promoter region in human colorectal carcinoma.

### Summary of the Invention

Methods of the invention make it possible to detect a disease in a biological sample without using an invasive procedure. According to the invention, by conducting an assay to detect methylation changes on a heterogeneous nucleic acid sample, such as a sample obtained from stool, small amounts of nucleic acids with abnormal methylation can be detected that are indicative of the presence of a disease, e.g., cancer or precancer, in a patient. In one aspect, methods of the invention involve detecting the presence of small amounts of hypermethylated nucleic acid in a heterogeneous sample containing large amounts of unmethylated nucleic acid, using highly sensitive and specific nucleic acid amplification reactions. In another aspect, methods of the invention involve detecting the presence of small changes in the amount of hypomethylated nucleic acid in a heterogeneous sample containing both methylated and unmethylated nucleic acid, using highly sensitive and specific nucleic acid amplification reactions. In further aspects, methods of the invention can detect either hypermethylation or hypomethylation at a combination of two or more target CpG markers or loci to improve the reliability of disease detection.

In one aspect, methods of the invention comprise the utilization of improved primers for detecting hypermethylation or hypomethylation in a target nucleic acid. In a preferred embodiment, methods of the invention comprise conducting a nucleic acid amplification using one ore more (preferably two) chimeric primers. A chimeric primer, for purposes of the invention, is a primer having a 3' portion with substantial sequence specificity with the target template to be amplified (a temple-specific portion) and a 5' portion that is referred to herein as a "non-specific portion" that does not hybridize with the target template. The 5' non-specific sequence preferably includes at least 5, and preferably fewer than or equal to 30 nucleotides. Also, the 5' non-specific portion preferably has a G-C content of at least 50%. In a particularly preferred embodiment, the 5' non-specific portion of both the forward and the reverse primers are the same. The primers may include DNA, RNA or a nucleic acid analog such as PNA. In a preferred embodiment, the target nucleic acid is isolated from a stool sample.

In another aspect, methods of the invention comprise the optimization of reaction conditions for PCR to detect abnormal methylation in a subpopulation of nucleic acids in a biological sample. In a preferred embodiment, methods of the invention comprise determining a first melting temperature for a primer in a PCR that amplifies a target nucleic acid, and conducting a PCR at a second melting temperature that is higher than the first melting temperature, using at least one chimeric primer. The melting temperature, sometimes called the annealing temperature, as referred to herein, means the temperature at which the strands of a nucleic acid hybrid are about 50% dissociated. The second melting temperature is preferably more than about 3°C higher than the first melting temperature. In a highly preferred embodiment, the second melting temperature is about 65°C.

In another aspect, the invention provides methods for screening a population to detect patients with indicia of cancer. In preferred embodiments, a plurality of target CpG containing regions are interrogated for the presence ofhypermethylation or hypomethylation. Preferably, one or more regions associated with the following genes are assayed in patient nucleic acid isolated from a heterogeneous biological sample such as stool: Adenomatous Polyposis Coli (*APC)*, hypermethylated in cancer-1 (*HIC1*), protein 14 (*p14*), protein 16 (*p16*), O(6)-methylguanine-DNA methyltransferase (*MGMT*), helicase-like transcription factor (*HLTF*), Msx2-interacting nuclear target protein 1 (*MINT1*), Msx2-interacting nuclear target protein 2 (*MINT2*), Msx2-interacting nuclear target protein 31 (*MINT31*), human mut-L homologue 1 (*hMLH1*), thrombospondin 1 (*THBS1*), metalloproteinase-3 (*TIMP3*), and insulin-like growth factor II (*IGF2*) regulatory regions. The presence of abnormal methylation in at least one of these regions is indicative of the presence of an indicium of cancer in the patient. Specifically, hypomethylation and loss of imprinting in IGF2 gene has been shown to indicate at least a higher risk for CRC. Hypermethylation of other, including the rest of the above-mentioned genes, have also been implicated in the onset of CRC. According to the invention, hypermethylation is an amount of methylation that is greater than that found in normal tissue or normal cells isolated from a healthy patient. In contrast, hypomethylation is an amount of methylation less than that found in normal tissue or normal cells isolated from a healthy patient. Accordingly, preferred methods of the invention include a negative control assay using nucleic acid from normal cells. In more preferred embodiments, methods of the invention also include a positive control assay using either hypermethylated or hypomethylated nucleic acid.

In a further aspect, methods of the invention are also useful to detect abnormal methylation in a substantially homogeneous sample such as a biopsy sample.

These and other advantages and aspects of the invention will be understood upon consideration of the following detailed description thereof.

### Brief Description of the Drawings

Figure 1 shows a schematic diagram of the chimeric primers used in the present invention.

Figure 2 shows a schematic representation of PCR amplification using chimeric primers.

### Detailed Description of the Invention

The invention provides methods for detecting abnormally methylated nucleic acid in a heterogeneous biological sample. Methods of the invention involve amplifying a subpopulation of abnormally methylated nucleic acid, preferably DNA, using PCR primers with high specificity. According to the invention, the target nucleic acid can be any nucleic acid of interest: human DNA, viral DNA, etc. The target nucleic acid can be a double-stranded DNA, single-stranded DNA, RNA, a nucleic acid analog, e.g., PNA, or a combination of any of the above.

In heterogeneous nucleic acid samples (e.g., stool), abnormally methylated target nucleic acid may be present in a small amount relative to normally methylated target nucleic acid. This represents a significant obstacle to detecting the abnormally methylated nucleic acid against the background of normally methylated nucleic acid, using typical nucleic acid amplification methods. According to the invention, small relative amounts of abnormally methylated nucleic acid can be detected using higher amplification efficiency. Preferably, the sensitivity of the reaction is preserved or enhanced and the specificity of the reaction is increased. For PCR using conventional primers, for example, amplification efficiency remains around 50%-60% for each thermal cycle, even under optimal conditions. Meanwhile, as the degree of sample heterogeneity increases, tolerance for background (e.g. signal generated by the presence of normal DNA) becomes increasingly lower. According to the invention, it is necessary to retain a sufficient signal to noise ratio to distinguish positive clinical samples from negative or control samples within an assay, and for retaining high confidence in the assay results. Therefore, methods of the invention require a heightened specificity in amplification-based detection assays in order to detect a small amount of abnormally methylated target nucleic acid against a background of normally methylated nucleic acid in heterogeneous biological samples. In certain embodiments where the indicium of disease is loss of genetic imprinting or loss of methylation, methods of the invention may aim to detect an abnormally high percentage of nucleic acid in the sample that are hypomethylated in comparison to, for example, samples from a control population or samples obtained from the same patient at a reference time point.

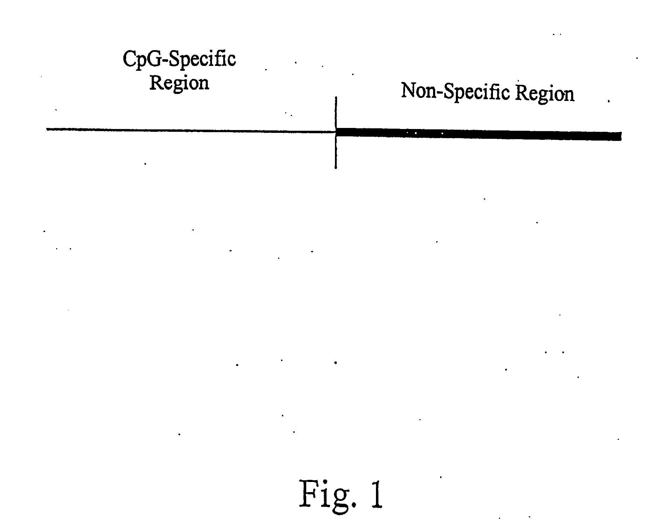

Referring to FIG. 1, according to the invention, a chimeric primer is provided that has a first portion that is not homologous or complementary to the target template ("non-specific portion" or "non-specific region"). The chimeric primer also contains a second, template-specific portion that is 3' to the first portion. The chimeric primer may consist of DNA, RNA, a nucleic acid analog, e.g., PNA, or a combination of any of the above.

The non-specific portion of the chimeric primer may be of any sequence, regardless of length, that does not have substantial sequence specificity (i.e., does not hybridize under stringent hybridization conditions) for any template sequence in an analyte sample. In a preferred embodiment, the non-specific portion is the 5' end of the chimeric primer. But there may be additional sequence 5' to the non-specific portion. To further prevent background binding, in a particular embodiment, the non-specific portion of the chimeric primer includes a high G-C content of at least 50%.

The template-specific portion is preferably complementary to or specific for a CpG-containing sequence on the target nucleic acid template. First, the differences between a mutant template that is abnormal in its methylation state and a wild type template are converted into sequence differences through chemical modifications of the templates as discussed in more detail in later parts of this application. Then, at least one chimeric primer with at least one template-specific portion is used to assay if the chemically-treated sample comprises a mutant template. In an embodiment for detecting hypermethylation, an amplification product will be produced and detected if the template in the sample comprises an unmodified cytosine at a predetermined position that matches with the template-specific portion of the primer. In another embodiment for detecting hypomethylation, an amplification product will be produced and detected if the template in the sample comprises a modified cytosine at a predetermined position that matches with the template-specific portion of the primer. In other words, for detection of hypermethylation, the template-specific portion of the primer is specific for a mutant sequence that is hypermethylated. Conversely, for detection of hypomethylation, the template-specific portion of the primer is specific for a mutant sequence that is hypomethylated. In a preferred embodiment, the template-specific portion may be specific for a plurality of CpG dinucleotides some or all of which may be in an abnormal methylation state, e.g., hypermethylated, while the rest of the CpG dinucleotides, whether methylated or not, are in their normal methylation state. The template-specific portion of the chimeric primer comprises a sequence that is complementary to a sequence (either unmodified or modified) on one strand of the target template. A template-specific portion of a chimeric primer that specifically hybridizes to an unmodified form of a sequence on the target template comprises a sequence that is complementary to an unmodified sequence on the target template. A template-specific portion of a chimeric primer that specifically hybridizes to a modified form of the same sequence comprises a sequence that is complementary to a modified form of that sequence on the target template. Preferably, the complementary sequence is at least 5 nucleotides long, more preferably between 5 and 15 nucleotides long, and even more preferably between 15 and 30 nucleotides long. In further embodiments, the sequence may be longer that 30 nucleotides. According to the invention, the chimeric primer may also contain a portion with a sequence that is partially complementary to the template sequence of interest (for example between 80% and 100% complementary), provided that the partially complementary portion does not interfere with the specificity of the chimeric primer. As described herein, a methylation specific chimeric primer includes one or more Gs (or analogues thereof) at predetermined positions complementary to one or more Cs (at predetermined CpG dinucleotides) that are suspected of being methylated (and consequently unmodified) on the target template strand that is being tested.

In preferred embodiments of the invention, the CpG-containing sequence useful as a target template is part of a promoter or regulatory region of a gene associated with cancer, such as a tumor suppressor gene. A preferred promoter region contains a plurality of CpG dinucleotides (CpG island(s)) that are abnormally methylated in cancerous cells, either hypermethylated or hypomethylated. However, since aberration in the methylation state genome-wide may be indicative of aberrant methylation in genes directly linked to a disease of interest, the template-specific portion of the primer may be specific for a region in the genome that is not part of a regulatory CpG island of a cancer associated gene. In more preferred embodiments of the invention, an amplification reaction is performed using a forward and a reverse chimeric primer, each of which includes a portion that is complementary to a different CpG containing sequence within the target nucleic acid region. Again, the target region is preferably, but not necessarily, part of a CpG island. In some embodiments, abnormal methylation can be detected in an amplification reaction that uses only one chimeric primer that includes a portion complementary to a CpG containing sequence. In this embodiment, amplification may be conducted using a second primer that includes a portion complementary to a non CpG containing sequence adjacent the target promoter or regulatory region. According to the invention, amplification may also be performed using one chimeric primer and one non-chimeric primer, provided that the chimeric primer includes a sequence that is complementary to a CpG containing sequence on the target nucleic acid.

The target nucleic acid template may be chosen to be part of a CpG island associated with some marker genes useful for diagnosing a disease of interest. For example, for diagnosis of CRC, any number of the following genes may be used for purpose of the invention: Adenomatous Polyposis Coli (*APC*), hypermethylated in cancer-1 (*HIC1*), protein 14 (*p14*), protein 16 (*p16*), O(6)-methylguanine-DNA methyltransferase (*MGMT*), helicase-like transcription factor (*HLTF*), Msx2-interacting nuclear target protein 1 (*MINT1*), Msx2-interacting nuclear target protein 2 (*MINT2*), Msx2-interacting nuclear target protein 31 (*MINT31*), human mut-L homologue 1 (*hMLH1*), thrombospondin 1 (*THBS1*), metalloproteinase-3 (*TIMP3*), and insulin-like growth factor II (*IGF2*).

When a chimeric primer as described above is used in a PCR, non-specific binding to the template is reduced because matching between the template-specific portion tail and a target nucleic acid has to be more exact to offset the apparent non-matching by the non-specific portion. In other words, under the same condition, a template-specific sequence has a lower tolerance for mismatches when it is part of a chimeric primer than by itself. The specificity is even higher when the melting temperature is elevated in a preferred embodiment of the invention.

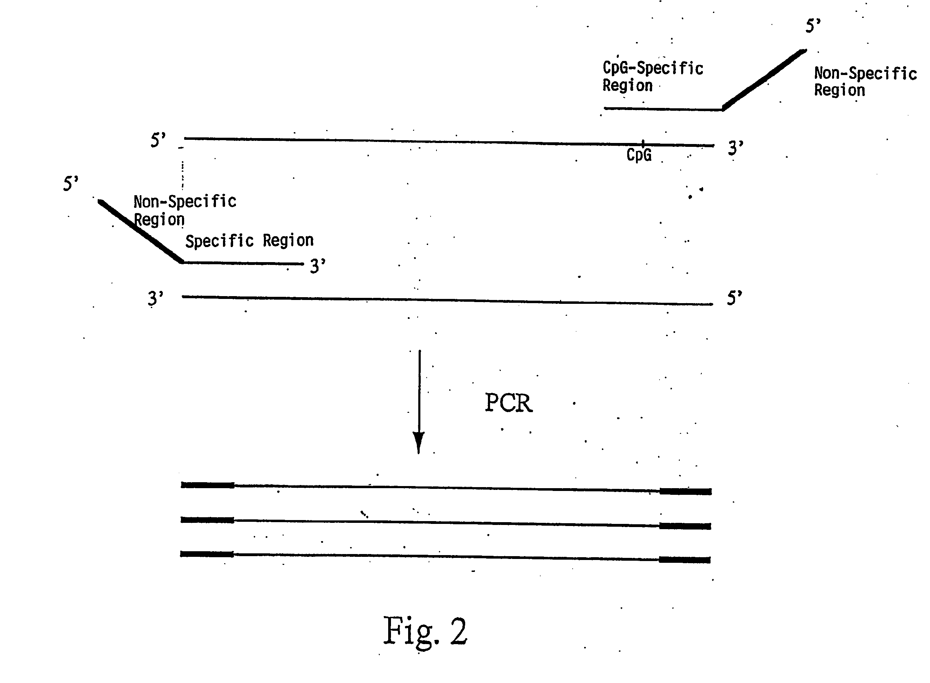

Conventional PCR efficiency typically does not exceed 60% for each thermal extension cycle. When a chimeric primer according to the present invention is used, the efficiency for the first round of extension in PCR may be further compromised (e.g., 20%). However, in subsequent PCR cycles where the chimeric primer is used, extension efficiency may reach as high as 100%. This makes methods of the present invention more efficient in amplifying a specific target template over multiple PCR cycles (e.g. 20 cycles). As a result, the number of PCR cycles needed to amplify a rare event target in a heterogeneous sample for detection or further analysis can be reduced by using a chimeric primer of the invention. While reasons for the enhanced extension efficiency in the later PCR cycles while using a chimeric primer are not fully understood, it has been postulated that the enhanced efficiency might be related to the fact that, following the first round of PCR involving a chimeric primer, the non-specific sequence becomes incorporated into the PCR products (amplicon) generated from the first round PCR, (See Fig. 2), and becomes part of a potential template for binding. Overall improvement in PCR efficiency is also observed when the melting temperature is elevated.

Accordingly, methods of the invention provide heightened specificity and efficiency in an amplification-based screening method that looks for abnormally methylated nucleic acid in a heterogeneous sample. According to preferred embodiments, an agent that modifies unmethylated cytosine without modifying methylated cytosine is added to a nucleic acid sample isolated from a heterogeneous biological sample. An amplification reaction is then performed using at least one chimeric primer that distinguishes between a modified and an unmodified CpG dinucleotide. Detection of an amplification product of unmodified CpG dinucleotide indicates the presence of methylated CpG dinucleotide, which may be hypermethylated if the wild type sequence is not or less heavily methylated. Conversely, detection of an amplification product of modified CpG dinucleotide indicates the presence of unmethylated CpG dinucleotide, which may be hypomethylated if the wild type sequence is or more heavily methylated. Both results could serve as indication of the presence of diseased cells in the sample.

According to the invention, a heterogeneous nucleic acid sample is isolated from a heterogeneous biological sample, for example, stool. A stool sample contains a highly-heterogeneous population of nucleic acids. Not only does human nucleic acid represent a small portion of the nucleic acid found in stool, but nucleic acid from cancer cells, if present, represents only a small portion of the human nucleic acid. A stool sample may contain molecular indicia of cancer, specifically, colorectal cancer, that occurs as a small subpopulation (typically on the order of about 1% at early stages of cancer or precancer) of the total human nucleic acid in the stool. Other heterogeneous biological samples may include urine, semen or seminal fluid, plasma serum, cerebrospinal fluid, pus, aspirate (e.g. breast nipple aspirate), pancreatic fluid, bile, lymph, saliva, vaginal fluid, amniotic fluid, sputum, blood, and other bodily fluids, secretions, or tissues that may or may not be mingled with other biological materials. These samples may contain nucleic acids that are indicative of a variety of diseases including cancers, e.g., prostate, breast, lung, thymus, ovarian, and so on.

According to the invention, target nucleic acid may be isolated from a heterogeneous biological sample using a sequence-specific hybrid capture step. The target template can be captured using a capture probe specific for the template. According to the invention, this hybrid capture step is preferably performed prior to CpG modification, and may result in a heterogeneous sample of target nucleic acid that contains both methylated and unmethylated forms of the target nucleic acid.

In the modification treatment, the nucleic acid sample is treated with an agent such as sodium bisulfate. Preferably, the agent modifies unmethylated cytosine to uracil without modifying methylated cytosine. Bisulfite modification treatment is described in U.S. Patent No. 6,017,704,

After the modification treatment, an amplification reaction is preformed with at least one chimeric primer. The chimeric primer contains a first portion that is not specific for the target template and a second portion that is 3' to the first portion. In one embodiment, the second portion is specific for a sequence containing at least one, two, three, four, five, or more than five unmodified or modified CpG dinucleotides. While the template-specific portion, i.e., the second portion of the chimeric primer preferably is specific for more than one CpG dinucleotide, the second portion of the primer may be specific for only one CpG dinucleotide as long as the assay result regarding that CpG dinucleotide is indicative of the disease. The amplification product may be any part of the genome, as genome-wide aberration in methylation state may be indicative of aberrant methylation in disease-causing genes. Preferably, the amplification product is part of a CpG island.

In a preferred embodiment, both forward and reverse primers are chimeric primers with respective template-specific portions that hybridize with unmodified CpG, hence, methylated sequences in the target template. These primers are termed "methylation specific primers" hereafter. The detection of an amplification product indicates methylation at the CpG sequence, preferably, hypermethylation at the CpG dinucleotide. These results may indicate the onset of a particular disease, e.g., a particular cancer.

In another embodiment, the second portion of the chimeric primer is specific for at least one CpG dinucelotide sequence modified by the bisulfite treatment, hence, non-methylated sequence. These primers are termed "non-methylation specific primers" hereafter. Since unmethylated cytosine is modified to uracil, it will be recognized as a thymine in PCR, and the primer can be constructed accordingly to contain a template-specific portion that recognizes the modified sequences. In a heterogeneous DNA sample, such a chimeric primer can be used to amplify a hypomethylated dinucleotide, preferably in a sequence related to a disease-indicating gene. For example, DNA carrying the IGF2 gene can be hybrid-captured and assayed using a non-methylation specific primer to detect the loss of imprinting and hypomethylation in the regulatory region of the IGF2 gene. The non-methylation specific primers primer may also be used as a positive control for the presence of DNA in the assay looking for indication of hypermethylation, since its amplification product indicates that there is at least one copy of target DNA that is not hypermethylated. According to the invention, a primer sequence that is specific for a methylated CpG dinucleotide (a CpG dinucleotide that is not modified by treatment with a modification agent) includes a G at the position that is complementary to the C of the CpG dinucleotide. In contrast, a primer sequence that is specific for an unmethylated CpG dinucleotide (a CpG dinucleotide that is modified by treatment with a modification agent) includes an A at the position that is complementary to the C of the CpG dinucleotide, because the C is modified to a U by the modification reaction. Conversely, for the other primer in the pair, if it contains any CpG dinucleotide in its sequence, the C should be changed to T for a non-methylation specific primer.

As described herein, the non-methylation specific primers have a higher A/T content than their corresponding methylation specific primers that are partly specific for a template that is the same except for the hypermethylation. According to the invention, in order to maintain a comparable specificity at a similar melting/annealing temperature, a few more nucleotides may be added to the sequence-specific portion of the non-methylation specific primers. For example, additional, preferably template-specific, nucleotides can be added 5' to the existing template-specific portion of the non-methylation specific primer. Because of other constraints in primer design such as concern over secondary structures, the template-specific portions of the non-methylation specific primers and the methylation specific primers for the same genetic marker can be designed to be specific for different regions in the template.

In an illustrative example of an assay method according to the invention, a heterogeneous DNA sample is first isolated from a biological sample (e.g., blood or stool) obtained from a patient. Three aliquots, A, B, and C, of the DNA sample are separately amplified by PCR. The PCR for aliquot A uses a conventional template-specific primer that will hybridize with a target sequence, and serves as a positive control (i.e., detection of amplicon from aliquot A indicates existence of target sequence in the sample). PCR for aliquot B uses a chimeric primer that has a 5' tail of non-specific sequence attached to the template-specific primer used in aliquot A. The melting temperature for the PCR for aliquot B may be elevated to 5°C higher than the melting temperature used for aliquots A and C. PCR for aliquot C uses a primer that includes the non-specific sequence itself as a negative control (i.e., detection of amplicon from aliquot C indicates that the results should be disregarded due to, e.g., contamination from previous PCR). The primer used for negative control may contain bases, preferably from one to nine, in addition to the non-specific sequence, as long as the added sequence does not hybridize to any target template in the sample.

The methods of the present invention are especially suitable for applications such as: clinical assays involving sporadic cancer detection (e.g. testing DNA from stool for colorectal cancer) and related kits; and other "rare event" clinical assays and related kits. Methods of the invention may also be used for infectious disease diagnostics and related kits, and inherited disease diagnostics and related kits.

An application of the invention is to screen a population of patients for a disease, e.g., CRC, with the following method: isolating a plurality of target nucleic acid templates from each patient, and then assay each of the template for presence of either hypermethylation or hypomethylation as described above. The presence of disease or risk of disease can be indicated if any one of the target templates shows a positive result of either hypermethylation or hypomethylation relative to the amount of hypermethylation or hypomethylation, respectively, observed in a control sample known to contain template only in its normal or reference state of methylation. In a preferred embodiment, the presence of a disease or a risk for a disease is indicated by the presence of altered methylation at a plurality of loci (e.g. two or more). Preferably, a plurality of templates are exposed to similar amplification reactions conditions, e.g., similar PCR temperatures using chimeric primers with similar melting temperatures.

### Example 1: DNA isolation and sequence-specific hybrid-capture

Stool samples were collected and prepared as described in U.S. Patent No. 5,741,650 and in co-pending applications Serial No. 08/876,638, filed June 16, 1997.
Specifically, stool was collected and prepared so that a sample contained at least a cross-sectional portion of a stool voided by a patient. Alternatively, whole stool may be used. Frozen stool samples were first homogenized in a physiologically compatible buffer (e.g., having a final concentration: 500 mM Tris, 16 mM EDTA and 10 mM NaCl, pH 9.0), using an Exactor II shaker for 15 minutes. A 20% SDS solution is added to a final concentration of 0.5%. Proteinase K is also added to a final concentration of 500 µg/ml and incubated at 37°C.

Following homogenization, each sample was centrifuged twice to remove particulate matter, and the supernatant was incubated with DNAse-free RNAse. DNA was then precipitated with 300 mM sodium acetate and isopropanol, centrifuged, and resuspended in a TE buffer (10 mM Tris, 1 mM EDTA, pH 7.4).

Following preparation of total nucleic acid, DNA including a target region of interest was captured using a target-sequence specific capture probe. For example, DNA molecules carrying one of the three human genetic markers described in Example 3 below were respectively captured using oligonucleotide capture probes specific for each target: p16, hMLH1, and MGMT. The specific capture probe for each of these target markers is listed in Table 1. Similarly, other capture probes can be used for capturing other target regions of interest. In the capture procedures, 300 µl of each sample DNA preparation was combined with an equal volume of 6M guanidinium isothiocyantate and 20 nmol of each sequence specific biotinylated oligonucleotide capture probe. The mixture was then heated to 95°C for 5 min, cooled on ice, and incubated overnight at 25°C. Following incubation, hybridized samples were diluted with 1 ml sterile H₂O, 100 µl (1 mg) paramagnetic polystyrene beads coated with streptavidin (Dynabeads® M-280 Streptavidin, Dynal ASA, Oslo, Norway) was added, and the sample-bead suspension was mixed continuously and incubated for one hour at room temperature. Following incubation, the bead bound biotin-oligonucleotide-DNA complexes were then isolated with a magnetic separator and washed four times with binding and washing buffer (B&W buffer; 1 M NaCl, 10 mM Tris (pH 7.2), 1 mM EDTA, and 0.1 % Tween® 20). Captured sequences were then eluted from the bead complexes by the addition of 40 µl 0.1X TE (1 mM Tris, 100 nM EDTA (pH 7.4)) and heated to 85°C for 4 minutes.

**Table 1. Capture probe sequences**

| | | |
|---|---|---|
| P16 capture probe | GGTGA CCAGC CAGCC CCTCC TCTTT CTTCC TCCGG TG | SEQ ID NO: 1 |
| MGMT capture probe | CGAGG CTGCC ACCGT CCCGA GGGAG AGCTC CGCAC TC | SEQ ID NO: 2 |
| hMLH1 capture probe | TCGTT TTTAG GAGCT CGGGG AATAC GAAAT ATCCA GCCAA | SEQ ID NO: 3 |

### Example 2: Sensitized detection of human APC promoter hypermethylation

Methods of the invention were used for detecting hypermethylation of CpG islands in the promoter region of human APC gene. Hypermethylation is indicative of abnormality or deficiency in *APC* protein's tumor suppression activity. The methods were shown to be highly sensitive in detecting small amounts of hypermethylated DNA in a heterogeneous sample.

DNA was first isolated and hybrid-captured from patients' stool samples as described in Example 1 except that the capture probe had the following sequence: CCCCG CCCAA CCGCA CAGCC TGCCT AGCCC TAGCC CC (SEQ ID NO: 4). The DNA sample then underwent bisulfite modification which converted unmethylated cytosines to uracil. Two aliquots of the bisulfite-treated samples were subjected to separate PCR. The primers used on the first aliquot were chimeric primers according to the invention, which included a 3' portion specific for a methylated target template; the primers used on the second aliquot were chimeric primers that included a 3' portion specific for an unmethylated-thus-modified target template. Amplification results from both aliquots were then subjected to gel electrophoresis, stained with ethidium bromide, and visualized.

Amplicon detected from the first aliquot is further purified and sequenced to confirm the positive result of hypermethylation. A high degree of specificity was observed for using methods of the invention in the detection assays.

Specific materials and methods for this example are as follows: DNA was modified by sodium bisulfite or other comparable agents as described in U.S. Patent 5,786,146 to Herman et al*.,* The bisulfite treatment modified unmethylated cytosine to uracil, which would be amplified as thymidine in the amplification product. The bisulfite treatment did not affect methylated cytosine, allowing subsequent PCR to distinguish between methylated DNA from unmethylated DNA in a heterogeneous population.

The chimeric primers used for amplifying the methylated target were 5'-GCGGT CCCAA AAGGG TCAGT TATTG CGGAG TGCGG GTC-3' for the forward primer (SEQ ID NO: 5) and 5'-GCGGT CCCAA AAGGG TCAGT TCGAC GAACT CCCGA CGA-3' for the reverse primer (SEQ ID NO: 6). The chimeric primers used for amplifying the unmethylated target, and therefore containing T (or its complement A) in place of C (or its complement G), were 5'-GCGGT CCCAA AAGGG TCAGT GTGTT TTATT GTGGA GTGTG GGTT-3' for the forward primer (SEQ ID NO: 7) and 5'-GCGGT CCCAA AAGGG TCAGT CCAAT CAACA AACTC CCAAC AA-3' for the reverse primer (SEQ ID NO: 8). All four primers have the same 5' end that is the non-specific portion: 5'-GCGGTCCCAAAAGGGTCAGT-3' (SEQ ID NO: 9). All the oligonucleotides used in the example were HPLC purified and quantified by spectrophotometry.

PCR for each aliquot was performed in a 50 µl reaction sample with 10 µl bisulfite-treated DNA. After Hot Star polymerase (Qiagen) was added, each reaction mixture was hot-started at 95°C for 15 minutes. The reactions were carried out in a Perkin-Elmer 9600 Thermal Cycler (Perkin-Elmer, Norwalk, CT) with the following temperature settings: 39 cycles of 94°C for 30 seconds, 65°C for 60 seconds, and 72°C for 60 seconds. The melting/annealing temperature of 65°C was 10°C higher than the conventional annealing temperature used in Esterller M., et al., "Analysis of Adenomatous Polyposis Coli Promoter Hypermethylation in Human Cancer," 60 Cancer Research, 4366 (Aug. 15, 2000), incorporated herein by reference.

For PCR product analyses, 8 µl of the amplification reactions was loaded directly onto a 4% ethidium bromide stained agarose gel and electrophoresed at 200 V for 90 min. The amplification products were visualized with a UV transilluminator (Fotodyne, New Berlin, WI) and photographed with an Alpha Innotech IS-500 Digital Imaging System version 1.97 (Sun Bioscience Inc., Branford, CT).

Positive results of hypermethylation, i.e., the amplicon from the first aliquot, was purified using Wizard PCR Preps DNA Purification System (Promega) and sequenced out for confirmation.

### Example 3: Detection of hypermethylation at multiple loci

Methods of the invention were used to screen for colorectal cancer by detecting hypermethylation of CpG islands at multiple genes or genetic loci using stool samples. Assaying for hypermethylation at multiple loci in a heterogeneous sample may provide a more reliable means of detection certain diseases.

Hypermethylation markers on three human genes p16, hMLH1, and MGMT were studied using methods of the invention. Tissue samples (colorectal mucosa) and single stools were each collected from 12 patients clinically diagnosed with colorectal cancers. Single stools were each collected from 10 colonoscopically normal controls. All stools were collected prior to cathartic cleansing for colonoscopy or surgery, and were promptly stored at -70°C.

DNA was isolated from collected tissues (colorectal mucosa) using conventional techniques (Qiagen DNA Mini Kit). DNA was isolated from collected stools and subjected to sequence-specific hybrid capture as described in Example 1 except that the hybrid-captured DNA was eluted from bead complexes directly into the alkaline solution for initiating the bisulfite modification. Bisulfite modification of DNA samples was carried out as described in Example 2. Afterwards, hypermethylated markers were assayed by PCR techniques of the invention using chimeric primers either partly specific for unmodified (hence methylated) CpG sequence, as listed in Table 2, or partly specific for modified (hence unmethylated) CpG sequence, as listed in Table 3. The DNA samples were analyzed in blinded fashion.

**Table 2. Methylation-specific primer sequences**

| Primer Description | Sequence | Tm for PCR | SEQ ID NO: |
|---|---|---|---|
| P16 PCR forward primer | GCGGT CCCAA AAGGG TCAGT GGCGG ATCGA GTGCG TTC | 58°C | 10 |
| P16 PCR reverse primer | GCGGT CCCAA AAGGG TCAGT ACTCC CCGCC GCCCG CTA | 58°C | 11 |
| hMLH1 PCR forward primer | GCGGT CCCAA AAGGG TCAGT ATTAA TAGGA AGAGC GGATA GC | 58°C | 12 |
| hMLH1 PCR reverse primer | GCGGT CCCAA AAGGG TCAGT TTCGT CCCTC CCTAA AACGA | 58°C | 13 |
| MGMT PCR forward primer | GCGGT CCCAA AAGGG TCAGT CGTTT TGCGT TTCGA CGTTC | 55°C | 14 |
| MGMT PCR reverse primer | GCGGT CCCAA AAGGG TCAGT CACTC TTCCG AAAAC GAAAC G | 55°C | 15 |

**Table 3. Non-methylation-specific primer sequences**

| Primer Description | Sequence | Tm for PCR | SEQ ID NO: |
|---|---|---|---|
| P16 PCR forward primer | GCGGT CCCAA AAGGG TCAGT GGGGT GGATT GAGTG TGTTT | 58°C | 16 |
| P16 PCR reverse primer | GCGGT CCCAA AAGGG TCAGT ATACT ACTCC CCACC ACCAA | 58°C | 17 |
| hMLH1 PCR forward primer | GCGGT CCCAA AAGGG TCAGT AATGA ATTAA TAGGA AGAGT GGATA GT | 58°C | 18 |
| hMLH1 PCR reverse primer | GCGGT CCCAA AAGGG TCAGT TCTCT TCATC CCTCC CTAAA ACA | 58°C | 19 |
| MGMT PCR forward primer | GCGGT CCCAA AAGGG TCAGT TTTTT AGAAT GTTTT GTGTT TTGAT | 55°C | 20 |
| MGMT P CR reverse primer | GCGGT CCCAA AAGGG TCAGT ACCCA AACAC TCACC AAATC A | 55°C | 21 |

For tissue/mucosa samples, at least one of the three markers was found to be hypermethylated in samples from 9 out of the 12 (75%) cancer patients. Individually, the p16 marker was positive in 7 cancer patients, hMLH1 in 3 cancer patents, and MGMT in 6 cancer patients. None of the markers was found to be methylated in any tissue/mucosa samples from the 10 control patients.

For stool samples, at least one of the three markers was found to be hypermethylated in samples from 7 out of the 12 (58%) cancer patients. Out of the 9 people identified positively through the tissue samples, 7 were identified positively using methods of the invention. Individually, the p 16 marker was positive in 4 cancer patients, MLH1 in 1 cancer patents, and MGMT in 4 cancer patients. The p16 marker was also positive in the stool sample of 1 control patient, and the MGMT marker in 2 of the control samples. These results may be an indicium of disease in patients considered to be healthy controls. The hMLH1 marker was not positive in any of the control patients.

Therefore, the invention provides non-invasive methods for detecting indicia of cancer in heterogeneous patient samples such as stool samples.

### Example 4: Alternative markers for detecting of hypermethylation

Other markers and sequences that may be useful in screening for colorectal cancer are also provided herein. Similar to Example 3, detection of hypermethylation in any number of the following genes may indicate a higher risk of colorectal cancer: p14, HLTF, MINT31, MINT2, HIC1. Any of these markers can be used in combination with any of the markers described in earlier examples, including APC, p16, hMLH1, and MGMT.

Examples of useful capture probes are provided in Table 4 below. Examples of chimeric primers partly specific for CpG sequences unmodified by the bisulfite treatment (hence methylated template) are provided in Table 5 below. Table 6 lists examples of chimeric primers partly specific for CpG sequences modified by the bisulfite treatment (hence unmethylated template).

**Table 4. Capture probe sequences**

| | | |
|---|---|---|
| P14 capture probe | CCTTA ACTGC AGACT GGGAC CCACG CACCG CCGCC TG | SEQ ID NO: 22 |
| HLTF capture probe | GGTGC CTGCG GTGCC GGTGT TTTGT TTGAT TCCCT GC | SEQ ID NO: 23 |
| MINT31 capture probe | ATGGG GGGGA GGGGA TGGGG GAAGT GGCAG AGTGG CT | SEQ ID NO: 24 |
| MINT2 capture probe | ACTTG GGTGC TAGAG TTCGG CGGGA AGAAA AGGTG AC | SEQ ID NO: 25 |

**Table 5. Methylation-specific primer sequences**

| **Primer Description** | **Sequence** | **SEQ ID NO.** |
|---|---|---|
| P 14 PCR forward primer | GCGGT CCCAA TAGGG TCAGT GTGTT AAAGG GCGGC GTAGC | 26 |
| P 14 PCR reverse primer | GCGGT CCCAA AAGGG TCAGT AAAAC CCTCA CTCGC GACGA | 27 |
| HLTF PCR forward primer | GCGGT CCCAA TAGGG TCAGT GTTTC GTGGT TTTTT CGCGC | 28 |
| HLTF PCR reverse primer | GCGGT CCCAA TAGGG TCATT CAAAA ACGAC GAAAA ATCCG | 29 |
| MINT31 forward primer | GCGGT CCCAA TAGGG TCAGT TTTTT TTTTA TCGGT CGGGG C | 30 |
| MINT31 reverse primer | GCGGT CCCAA TAGGG TCAGT CCTCC CGCCG CCGCA AAA | 31 |
| MINT2 forward primer | GCGGT CCCAA TAGGG TCAGT TTTGT TAAAG TGTTG AGTTC GTC | 32 |
| MINT2 reverse primer | GCGGT CCCAA TAGGG TCAGT CGAAA TAATA ACGAC GATTC CG | 33 |

**Table 6. Non-methylation-specific primer sequences**

| **Primer Description** | **Sequence** | **SEQ ID NO.** |
|---|---|---|
| P14 PCR forward primer | GCGGT CCCAA AAGGG TCAGT TTTTT GGTGT TAAAG GGTGG TGTAG T | 34 |
| P 14 PCR reverse primer | GCGGT CCCAA AAGGG TCAGT CACAA AAACC CTCAC TCACA ACAA | 35 |
| HLTF PCR forward primer | GCGGT CCCAA TAGGG TCAGT GGGGT TTTGT GGTTT TTTTG TGT | 36 |
| HLTF PCR reverse primer | GCGGT CCCAA TAGGG TCAGT AAAAA AACAA CTCCA CCCCA CTA | 37 |
| MINT31 PCR forward primer | GCGGT CCCAA TAGGG TCAGT TTTTT TTTTT TTTAT TGGTT GGGGT | 38 |
| MINT31 PCR reverse primer | GCGGT CCCAA TAGGG TCAGT CACCC ACACA ACCCC CACA | 39 |
| MINT2 PCR forward primer | GCGGT CCCAA TAGGG TCAGT GTTGG TGGAT TTTGG ATTTT GTT | 40 |
| MINT2 PCR reverse primer | GCGGT CCCAA TAGGG TCAGT CCAAA ATAAT AACAA CAATT CCATA | 41 |

Exemplary materials and methods are as follows: tissue samples and stool samples were first collected. Then the stool sample was processed to extract a DNA sample as described in Example 1 up to the step of isolating bead bound biotin-oligonucleotide-DNA complexes with a magnetic separator. After the separated beads had been washed, bound DNA was eluted in 40 µL of an elution buffer (0.3 N NaOH and 0.5 µg Salmon Sperm DNA) and vortexed gently. After standing at room temperature for 5 minutes, the sample was briefly vortexed again. The beads were separated by the magnetic separator and the supernatant was transferred to a 1.5 ml microcentrifuge tube. After the supernatant had been incubated at 37°C for 10 minutes, 30 µl of freshly prepared 10 mM hydroquinone and 500 µl of freshly prepared 3M sodium bisulfite (pH 5.0) were added to the supernatant and vortexed. After 200 µl of mineral oil was added to the combination, the sample was spun briefly and incubated at 50°C overnight.

DNA molecules extracted from the collected tissue sample were processed as follows: 12 µl of 1N NaOH and 0.5 µl salmon sperm DNA (1.0 µg/µl) were mixed with 1 to 300 ng of tissue DNA to bring the total volume to 40 µl for each sample. After the sample had been incubated at 37°C for 10 minutes, 30 µl of freshly prepared 10 mM hydroquinone and 500 µl of freshly prepared 3M sodium bisulfite (pH 5.0) were added to the sample and vortexed. After 200 µl of mineral oil was added to the combination, the sample was spun briefly and incubated at 50°C overnight.

After the bisulfite treatment, both the stool DNA and tissue DNA samples were purified using Wizard columns (Promega) and used for PCR using one or more of the primers listed in Tables 2, 3, 5, and 6. For each PCR reaction, an exemplary sample included 10 µl bisulfite treated DNA and 40 µl of a master mix. The master mix consisted of 24.5 µl of sterile water, 5 µl of Hot Star 10X buffer, 5 µl of 2 mM dNTP, 5 µl of 5 µM of the primer and 0.5 µl of Hot Star Taq DNA polymerase.

PCR reactions were carried out on a Tetrad® Cycler (MJ Research, Inc.) or other compatible equipment. In general, a PCR sample was first denatured at 95°C for 14.5 minutes, and then subjected to 40 cycles of the following: 94°C denaturation for 30 seconds, and one minute of annealing at various temperatures depending on the primer used (see Table 7 for details), and one minute of elongation at 72°C. After the 40 cycles, the sample underwent another 5 minutes of elongation at 72°C.

**Table 7: Annealing temperatures in PCR with various primers**

| Target Marker | Primer specific for | Annealing temperature (°C) |
|---|---|---|
| APC | Methylation | 65 |
| | Non-methylation | 60 |
| hMLH1 | Methylation | 58 |
| | Non-methylation | 58 |
| p16 | Methylation | 58 |
| | Non-methylation | 58 |
| MGMT | Methylation | 55 |
| | Non-methylation | 55 |
| HIC1 | Methylation | 60 |
| | Non-methylation | 60 |
| p14 | Methylation | 60 |
| | Non-methylation | 60 |
| HLTF | Methylation | 60 |
| | Non-methylation | 60 |
| MINT31 | Methylation | 60 |
| | Non-methylation | 60 |
| MINT2 | Methylation | 62 |
| | Non-methylation | 55 |

PCR products were visualized through agarose gel electrophoresis (4% NuSieve in 1X TAE buffer). The amount ofmethylation specific product relative to non-methylation specific product can be compared to that obtained for a known reference sample to determine whether the amount of methylation in the test sample is above normal.

## Claims

1. A method for determining the presence of a methylated CpG in a nucleic acid, said method comprising the steps of:
isolating a heterogeneous nucleic-acid sample comprising a CpG-containing target nucleic acid from a heterogeneous biological sample;
contacting said nucleic-acid sample with an agent that modifies unmethylated cytosine;
performing an amplification reaction on said target nucleic acid using at least one chimeric primer that comprises a first portion that is not specific for said target nucleic acid and a second portion 3' to said first portion, said second portion being specific for a methylated cytosine in said target nucleic acid; and
detecting the presence of an amplification product to determine the presence of a methylated CpG in the target nucleic acid, and
wherein the amplification reaction is a PCR reaction in which the non-specific sequence of a chimeric primer becomes incorporated into a PCR product after a first round of PCR.

2. The method of claim 1, wherein the isolating step comprises sequence-specific hybrid capture.

3. The method of claim 1, wherein said first portion comprises at least 5, but fewer than or equal to about 30 nucleotides.

4. The method of claim 1, wherein said second portion comprises from about 10 to about 30 nucleotides.

5. The method of claim 1, wherein at least two chimeric primers are used, and wherein each of said at least two primers has an identical first portion.

6. The method of claim 1, wherein said first portion has a G-C content of at least 50%.

7. The method of claim 1, wherein said target nucleic acid is selected from the group consisting of DNA, RNA, and a nucleic acid analog.

8. The method of claim 1, wherein said target nucleic acid is double-stranded.

9. The method of claim 1, wherein said target nucleic acid is at least a portion of a gene selected from the group consisting of Adenomatous Polyposis Coli (APC) gene, p16, hMLH1, MGMT, H1C1, p14, HLTF, MINT2, and MINT31.

10. Use of the method of claim 1 for screening a population of patients for an indicium of colorectal cancer or precancer, the method comprising the steps of:
providing a plurality of target nucleic acid templates isolated from each patient; and
assaying each of said templates for the presence of hypermethylation according to the method of claim 1.

11. The method according to any one of the preceding claims, wherein the heterogeneous biological sample is a stool sample.

12. The method according to any one of the preceding claims, wherein said target nucleic acid is at least a portion of a human Adenomatous Polyposis Coli (APC) gene.

13. The method according to any one of any one of the preceding claims, wherein said target nucleic acid is at least a portion of a HLTF gene.

## Patentansprüche

1. Verfahren zum Feststellen des Vorhandenseins eines methylierten CgG in einer Nukleinsäure, wobei das Verfahren folgende Schritte aufweist:
Isolieren einer heterogenen Nukleinsäureprobe, die eine CpG-haltige Zielnukleinsäure aufweist, aus einer heterogenen biologischen Probe;
Kontaktieren der Nukleinsäureprobe mit einem Mittel, das unmethyliertes Cytosin modifiziert;
Ausführen einer Amplifikationsreaktion an der Zielnukleinsäure unter Verwendung von mindestens einem chimären Primer, der einen ersten Teil,
welcher für die Zielnukleinsäure nicht spezifisch ist, und einen zweiten, 3'-Teil an dem ersten Teil aufweist, wobei der zweite Teil für ein methyliertes Cytosin in der Zielnukleinsäure spezifisch ist; und
Detektieren des Vorhandenseins eines Amplifikationsprodukts, um das Vorhandensein eines methylierten CpG in der Zielnukleinsäure festzustellen, und
wobei es sich bei der Amplifikationsreaktion um eine PCR-Reaktion handelt, bei der die nicht spezifische Sequenz eines chimären Primers nach einer ersten PCR-Runde in ein PCR-Produkt integriert wird.

2. Verfahren nach Anspruch 1,
wobei der Isolierschritt einen sequenz-spezifischen Fangvorgang aufweist.

3. Verfahren nach Anspruch 1,
wobei der erste Teil mindestens 5, jedoch weniger als oder gleich ca. 30 Nukleotide aufweist.

4. Verfahren nach Anspruch 1,
wobei der zweite Teil von ca. 10 bis ca. 30 Nukleotide aufweist.

5. Verfahren nach Anspruch 1,
wobei mindestens zwei chimäre Primer verwendet werden und wobei jeder der mindestens zwei Primer einen identischen ersten Teil aufweist.

6. Verfahren nach Anspruch 1,
wobei der erste Teil einen G-C-Gehalt von mindestens 50 % aufweist.

7. Verfahren nach Anspruch 1,
wobei die Zielnukleinsäure aus der Gruppe ausgewählt wird, die aus DNA, RNA und einem Nukleinsäure-Analog besteht.

8. Verfahren nach Anspruch 1,
wobei die Zielnukleinsäure doppelsträngig ist.

9. Verfahren nach Anspruch 1,
wobei es sich bei der Zielnukleinsäure zumindest um einen Teil eines Gens handelt, das aus der Gruppe ausgewählt ist, die aus dem adenomatösen Polyposis Coli- (APC-) Gen, p16, hMLH1, MGMT, H1C1, p14, HLTF, MINT2 und MINT31 besteht.

10. Verwendung des Verfahrens nach Anspruch 1 für das Screening einer Population von Patienten auf ein Anzeichen von Darmkrebs oder einer Krebsvorstufe, wobei das Verfahren folgende Schritte aufweist:
Bereitsstellen einer Mehrzahl von Zielnukleinsäure-Matrizen, die von jedem Patienten isoliert werden; und
Prüfen jeder der Matrizen auf das Vorhandensein von Hypermethylierung nach dem Verfahren gemäß Anspruch 1.

11. Verfahren nach einem der vorausgehenden Ansprüche,
wobei es sich bei der heterogenen biologischen Probe um eine Stuhlprobe handelt.

12. Verfahren nach einem der vorausgehenden Ansprüche,
wobei es sich bei der Zielnukleinsäure zumindest um einen Teil eines menschlichen adenomatösen Polyposis Coli- (APC-) Gens handelt.

13. Verfahren nach einem der vorausgehenden Ansprüche,
wobei es sich bei der Zielnukleinsäure zumindest um einen Teil eines HLTF-Gens handelt.

## Revendications

1. Procédé pour déterminer la présence d'un CpG méthylé dans un acide nucléique, ledit procédé comprenant les étapes :
d'isolement d'un échantillon d'acide nucléique hétérogène comprenant un acide nucléique cible contenant un CpG à partir d'un échantillon biologique hétérogène ;
de mise en contact dudit échantillon d'acide nucléique avec un agent qui modifie la cytosine non méthylée ;
de réaction d'amplification sur ledit acide nucléique cible en utilisant au moins une amorce chimère qui comprend une première partie qui n'est pas spécifique dudit acide nucléique cible et une deuxième partie en 3' de ladite première partie, ladite deuxième partie étant spécifique d'une cytosine méthylée dans ledit acide nucléique cible ; et
de détection de la présence d'un produit de l'amplification pour déterminer la présence d'un CpG méthylé dans l'acide nucléique cible, et
dans lequel la réaction d'amplification est une réaction PCR dans laquelle la séquence non spécifique d'une amorce chimère est incorporée dans un produit de PCR après un premier cycle de PCR.

2. Procédé selon la revendication 1, dans lequel l'étape d'isolement comprend une capture d'hybride à spécificité de séquence.

3. Procédé selon la revendication 1, dans lequel ladite première partie comprend au moins 5, mais moins de 30 ou environ 30 nucléotides.

4. Procédé selon la revendication 1, dans lequel ladite deuxième partie comprend d'environ 10 à environ 30 nucléotides.

5. Procédé selon la revendication 1, dans lequel au moins deux amorces chimères sont utilisées, et dans lequel chacune desdites au moins deux amorces a une première partie identique.

6. Procédé selon la revendication 1, dans lequel ladite première partie a une teneur en G-C d'au moins 50 %.

7. Procédé selon la revendication 1, dans lequel ledit acide nucléique cible est choisi dans l'ensemble constitué par un ADN, un ARN, et un analogue d'acide nucléique.

8. Procédé selon la revendication 1, dans lequel ledit acide nucléique cible est bicaténaire.

9. Procédé selon la revendication 1, dans lequel ledit acide nucléique cible est au moins une partie d'un gène choisi dans l'ensemble constitué par le gène de la polypose adénomateuse colique (APC), p16, hMLH1, MGMT, H1C1, p14, HLTF, MINT2 et MINT31.

10. Utilisation du procédé de la revendication 1 pour dépister, chez une population de patients, un indice de cancer ou de pré-cancer colorectal, le procédé comprenant les étapes consistant à :
se pourvoir d'une pluralité de matrices d'acides nucléiques cibles isolées à partir de chaque patient ; et
tester chacune desdites matrices pour déterminer la présence d'une hyperméthylation conformément au procédé de la revendication 1.

11. Procédé selon l'une quelconque des revendications précédentes, dans lequel l'échantillon biologique hétérogène est un échantillon de selles.

12. Procédé selon l'une quelconque des revendications précédentes, dans lequel ledit acide nucléique cible est au moins une partie d'un gène de la polypose adénomateuse colique (APC).

13. Procédé selon l'une quelconque des revendications précédentes, dans lequel ledit acide nucléique cible est au moins une partie d'un gène HLTF.
